# EUROPEAN PATENT SPECIFICATION

(11) **EP 2 575 085 B1**
(45) Date of publication and mention of the grant of the patent: **22.07.2015**
(21) Application number: 11007954.8
(22) Date of filing: 30.09.2011
(51) Int. Cl.: G06K 19/077, G06K 17/00, G06K 7/10

(54) **System, method and product for managing interactions of a person with objects**
System, Verfahren und Produkt zur Verwaltung von Interaktionen zwischen einer Person mit Objekten
Système, procédé et produit pour gérer les interactions d'une personne avec des objets

(43) Date of publication of application: 03.04.2013
(73) Proprietor: Fundacion Universidad San Jorge, 50830 Villanueva de Gallego Zaragoza (ES)
(72) Inventor: Ezquerra Diaz, Susana, 22005 Huesca (ES); Cetina Englada, Carlos, 50820 Zaragoza (ES); Friginal Santos-Olmo, Moises, 22005 Huesca (ES)
(74) Representative: Carvajal y Urquijo, Isabel

(56) References cited:
- WO-A2-2007/146497

## Description

### Field of the invention

The present invention pertains to the field of computer assistance and management of events associated with personal interactions with objects of his environment. Specifically, the invention is also related to applications for memory aid.

### Background of the invention

At the moment, the state of the art provides several examples related to the subject matter of the present invention. Mainly, they relate to installation of sensors in places where elderly people or problem memory patients live. These sensors supervise person's behaviour for identifying evidences of a worsened ability for remembering or medical emergency situations.

Hayes, T., Hunt, J., Adami, A. and Kaye, J. "An electronic pillbox for continuous monitoring of medication adherence" Proc. EMBS, (2006), 6400-3. The document proposes to include a sensor in medicine packages in order to check whether the patient observes the prescribed dosage and posology.

Matthew L. Lee and Anind K. Dey. 2011. "Reflecting on pills and phone use: supporting awareness of functional abilities for older adults". Proceedings of the 2011 annual conference on Human factors in computing systems (CHI '11). ACM, New York, NY, USA, 2095-2104. This paper proposes to install sensors in medicine packs as well as in home phones with the purpose of detecting changes in patient's habits that may indicate his memory is worsening.

As discussed above, the documents are focused on the user interaction with particular situations and do not address the complexity of the problem when there are more than one type of object to interact with. By contrast, according to the present invention, different objects are considered and likewise the possible relationships amongst them can be inferred.

Lee, M.L. and Dey, A.K. 2008. "Lifelogging Memory Appliance for People with Episodic Memory Impairment" Proceedings of the 10th International Conference on Ubiquitous Computing, 44-53. The authors propose to equip memory-problem patients with a device for taking random pictures daily. Then, such pictures are used to improve memory skills by questioning the patient to remember about what he was doing at those moments. As apparent, this approach is different for it is not focused on directly controlling effects of memory loss. Instead, the proposal intends to assist the patient in a treatment for rehabilitating his memory.

WO 2007/146497 A2 discloses a system of electronically managing interactions in accordance with the preamble of claim 1.

The present invention overcomes these shortcomings and obtains further advantages, which will become clear from the following sections.

### Summary of the invention

One of the purposes of the invention is to assist the user to seek and recover not aware information, i.e. because it is not available in his memory.

The proposed system retrieves information without requiring user's assistance when he is interacting with objects in an ordinary way. Advantageously, there are not intrusive actions when the invention is functioning.

Another improvement of the present invention is that the system setup and installation is very simple and can be completed easily.

The purpose is achieved using the system of claim 1, the method of claim 10 and the computer program of claim 13.

### Brief description of the drawings

Embodiments of the present invention will now be described, by way of example, with reference to the accompanying drawings in which:
FIG 1 shows a block diagram of the four main components of an embodiment of the invention, a reader (1) for reading a tag (2) of an object (4) and a mobile device (3) to which the reader (2) communicates the information retrieved from the tag (1) to be processed in order to find affinities for the object (4).
FIG 2 shows an example of a possible reader and its elements, namely, an antenna (10), a RF reader (11) and a RF transmitter (12).
FIG 3 shows an example of a mobile device (3) to process the information received from the reader (2). It includes a RF reader (31), a clock (22), a GPS receiver (23), a CPU (21) and a storage memory (24).
FIG 4 shows an example table of information retrieved by the mobile device 3, the Digital Object Identifier (DOI) as read from tag 1; Timestamp as read from a real-time clock 22; and Location with latitude and longitude coordinates as read from GPS receiver 23.
FIG 5A, 5B and 5C show a graph representing clusters of interaction points, where X-axis is time and Y-axis is space. FIG 5A illustrates a collection tag reading events for 3 different RFID Tags. FIG 5B illustrates the detection of clusters to assist in defining interactions. FIG 5C illustrates graphically how intersections of temporal and spatial intervals between clusters representing interactions are used to identify the existence of a temporal affinity and a spatial affinity.
FIG 6 is an example table of information generated by statistical clustering operations. As illustrated in table of FIG 6, the interaction points are grouped to form a cluster.
FIG 7 is an example table of information generated by affinity analysis on interactions.
FIG 8 shows a diagram representing a particular case with two different objects during several situations and the corresponding interaction points established according to the user's interactions.
FIG 9 shows an example of the steps that this system performes to generate interactions and affinities.
FIG 10 shows an example of possible uses of the information in storage 24.

### Preferred embodiment of the invention

For the purpose of better understanding the principles of the invention, reference is made to the embodiments illustrated in the drawings. Specific language may be used to describe the same without implying any limitation of the scope of the invention.

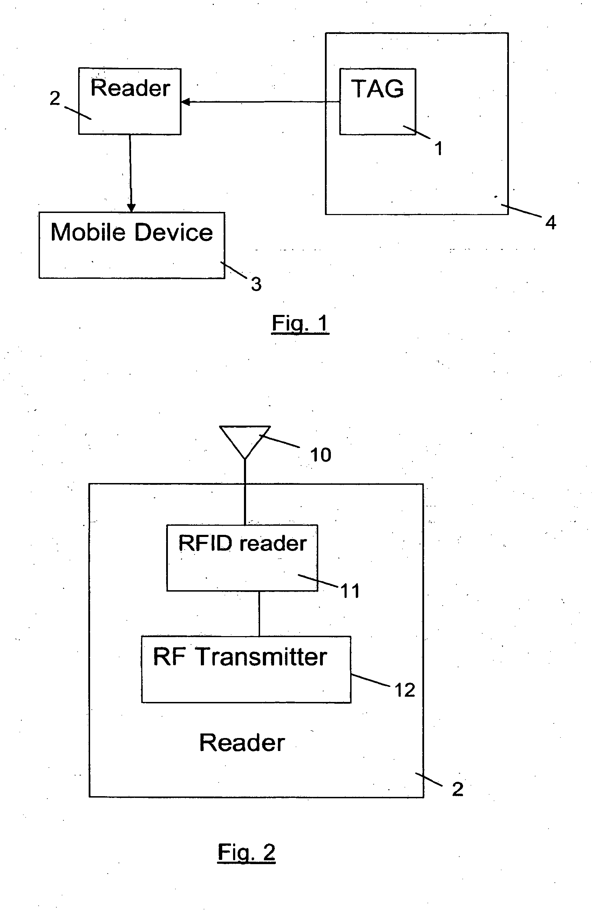

Fig 1 shows the main components and their interactions of the invention. RFID or NFC tags (1) are attached to a plurality of objects (4) in the environment. A portable reader (2) is worn by the person, preferably in or near his hand since most of the interactions of the person with the objects of his environment involve this part of the body. Other parts of the body are nevertheless possible.

When the user touches or approaches the portable reader (2) to the electromagnetic tag (1), its ID is read and sent to the mobile device (3), preferably using a standard wireless communication protocol, such as the Bluetooth protocol or any other low power RF transmission.

In this embodiment, the mobile device (3) and the reader (2) are separate components, forming different units. However, it is also possible to include both under the same device. Especially, this alternative may be advantageous when they are miniaturised.

The information stored on the electromagnetic tag (1) is a universally unique identification code (ID), different for each object, unlike other identification methods like bar codes that code families of objects. Each object of interest to the user must be supplied with an RFID tag (1) attached to the position in the object (4) where there are more possibilities to be touched, like the knob of a door, the handle of a pan or the key ring, because one of the aims of the invention is to be as unobtrusive as possible.

In this regard, under certain circumstances, it may be advisable that the user does not require to touch the object but rather to be close to it. This may depend on the type of object (i.e. a painting). Thus, other embodiments of the invention may be configured to be activated upon touching or in close proximity as well. Accordingly, one possible construction of portable reader (2) for this invention may include an RFID reader (11) which is equipped with an antenna (10) to increase the range of the interaction of the reader with the electromagnetic tag (1), this is shown in FIG 2. RFID power and antenna size are calculated according to common knowledge to limit interactions with other tags in the proximity area of the portable reader (2). The identification code read by the RFID reader (11) is transmitted via a low-power RF transmitter (12) module using some standard communication protocol, such as Bluetooth, but not limited to this protocol. Preferably, reader (2) is powered by a small battery (not shown) to make it portable and light-weight. One possible way to integrate all components is in a small wristband attached to the dominant arm of the user.

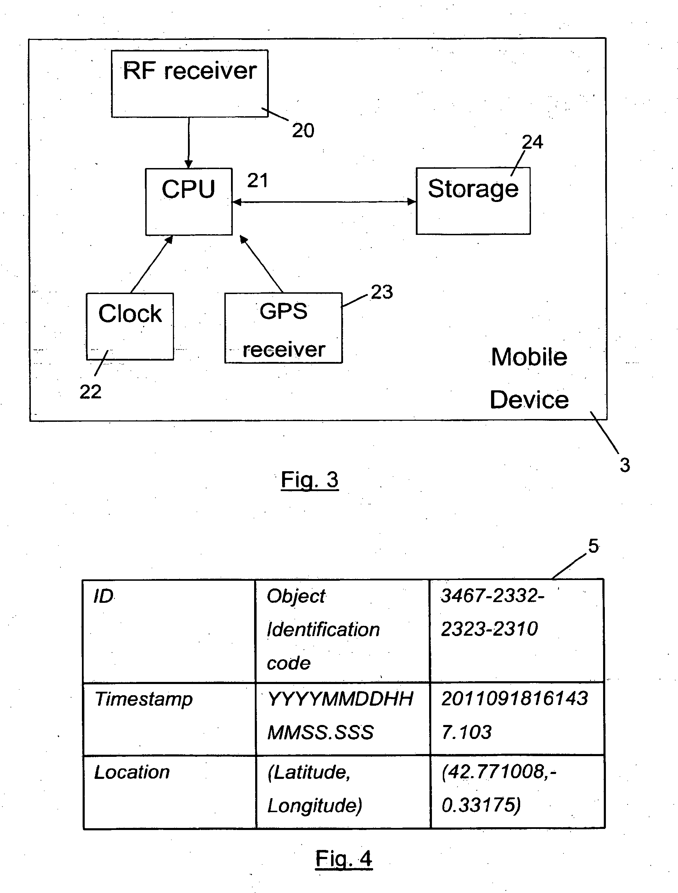

Fig. 3 shows a possible construction of mobile device (3) for this invention. Mobile device 3 gets the unique identification code of an object through an RF receiver (20) as it has been already explained. The time of the reception is obtained from a clock (22).

In an alternative embodiment of the invention, not shown on any figure, a clock (22) can be a component of a portable reader (2), which transmits the identification code of the electromagnetic tag (1) and the time of the interaction to the mobile device (3).

The location (longitude and latitude in geographic coordinates) of the mobile device can be obtained from a GPS receiver (23). This information is captured by the software running on CPU (21) of the mobile device (3) and stored persistently on storage (24), which may be a hard drive (HD), SD card, or any other type of non-volatile digital storage medium. Similarly, other embodiments may include a non-passive tag with spatial information therein. Also, temporal information may be present in the tag to be transmitted together with the identification.

As the user interacts with the objects of his environment, portable reader (2) begins to collect IDs from electromagnetic tags (1). Many readings are possible for the same object (4), if the user holds the object while moving around, or if portable reader (2) is configured to read several times per minute. A tag reading event (5) is stored in storage (24) of the mobile device (3) for each such reading, together with the time and location of the interaction, as provided by clock (22) and GPS receiver (23). Figure 4 shows one possible example of information stored in storage (24) for one tag reading event (5).

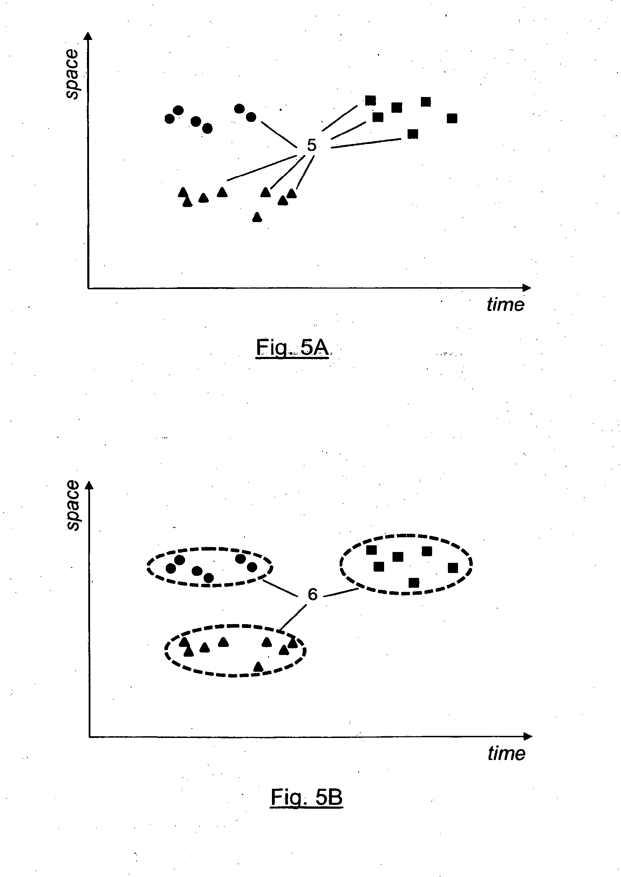

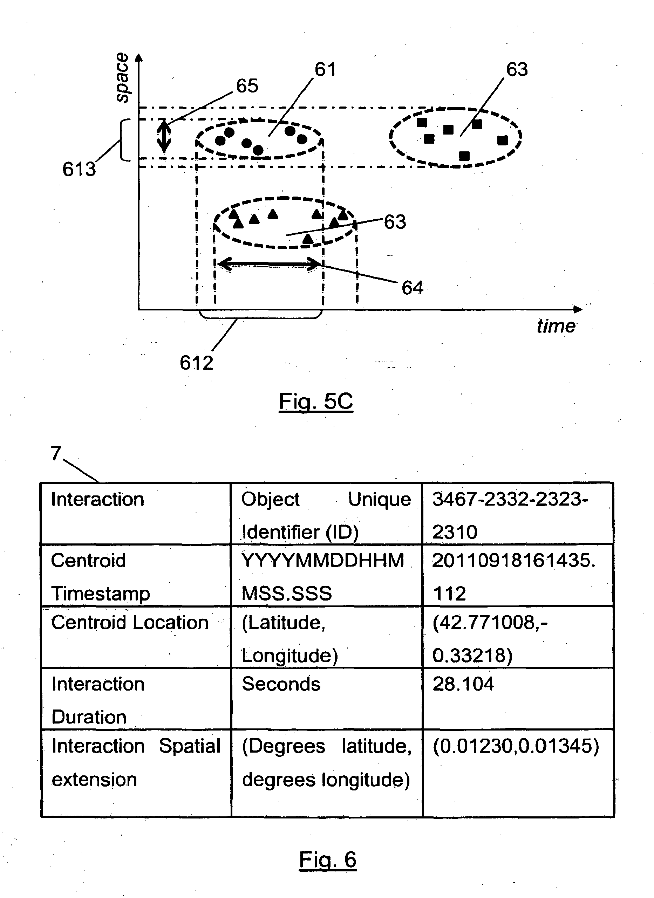

Figures 5A, 5B, and 5C show several graphs with time in the horizontal axis and space as a single dimension in the vertical axis with examples of information read or calculated by the system and stored in storage (24).

Figure 5A represents an example of tag reading events (5) for three different objects (4), each one with its unique ID. Tag reading events for the same object grouped together can be thought as pertaining to a unique interaction of the person with the object, with a temporal interval and a location area. This temporal interval and location area are identified by the system to characterize those objects that the person interact with and that occur at the same temporal interval and/or location area.

Figure 5B represents the clusters (6) of points identified by means of, for example but not excluding, a statistical clustering algorithm based on distance between tag reading events (5) such as k-means. The information supplied by this method is the centroid of the cluster, which is the tag reading event (5) representative of the interaction, together with an estimation of the time interval and spatial boundary of the interaction.

Figure 6 shows the information generated by clustering analysis for an example interaction (7). This information is stored in storage (24) for use in services offered to the user by mobile device (3). This information is a summary of the information contained in the tag reading events (5) for a single interaction of a person with one object.

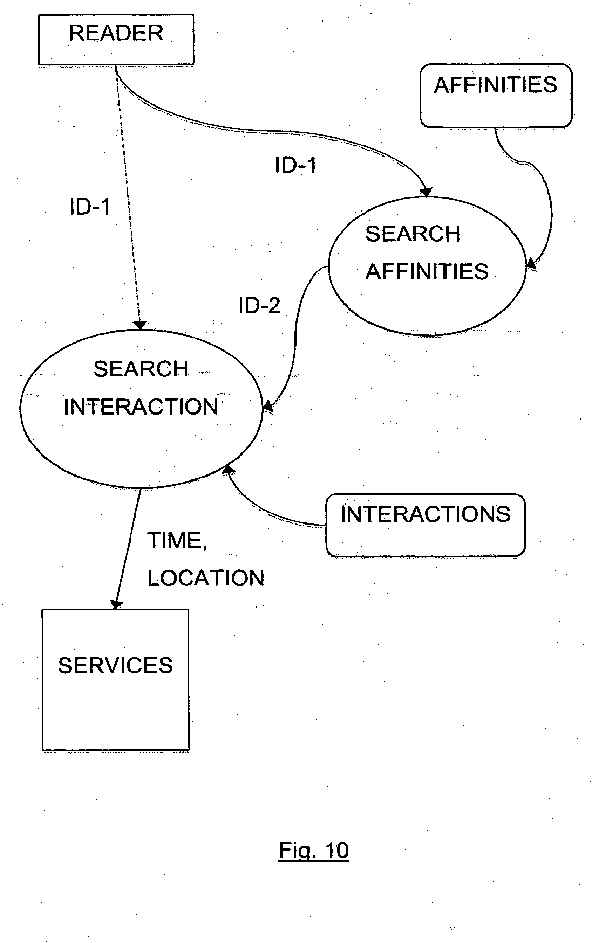

Figure 10 shows the process followed by a person that wants to receive information about his interactions with a certain object. This invention assumes that the person is not able or required to identify or remember the ID of the object. The process begins when the person uses portable reader (2) to touch the object (4). Reader (2) reads the ID-1 in the tag (1) and sends it to mobile device (3), this is shown as the dotted line in Fig 10. The user then begins a search in mobile device (3) for the interactions of ID-1 stored in storage (24), receiving a list with the time and location of the centroids of each interaction (7) with the same ID-1. As examples of services that use this list, it can be ordered by time, so the user can know the first or the last interaction with said object. Or the location of the interactions can be shown superimposed over a map.

This system can therefore find information about the interactions with objects without the need for the user to previously identify or remember the tag (1) attached to a particular object (4).

When the user has not access to the object (4) to launch this kind of services by reading its tag (1), for example, if the object has been lost or the user does not remember the last location where he left the object, this system can find this information by means of the relationships of objects in the environment. For example, if the user does not remember where he parked his car, he will surely hold the key of the car while he tries to remember the parking location. Because the key of the car and the car are used mostly together, their interactions stored in mobile device (4), from the tags (1) attached to these objects, will occur at similar times and locations. The system can calculate these similarities by means of affinity analysis.

Figure 5C shows the same example interactions and how the intersection of the temporal or spatial extensions of the cluster can lead to the establishment of affinities between different lDs. The system can determine the degree of spatial affinity between two interactions based on the existence of a common location area (65) for the clusters (6). To calculate the spatial span of the cluster, we use the location of the centroid plus the spatial extension of the cluster as calculated by clustering analysis and available in storage (24). The system can similarly determine the degree of temporal affinity between two interactions based on the existence of a simultaneous temporal interval (64) for the clusters. To calculate the temporal span of the cluster, we use the temporal coordinate of the centroid plus the temporal extension of the cluster as calculated by clustering analysis and available in storage (24).

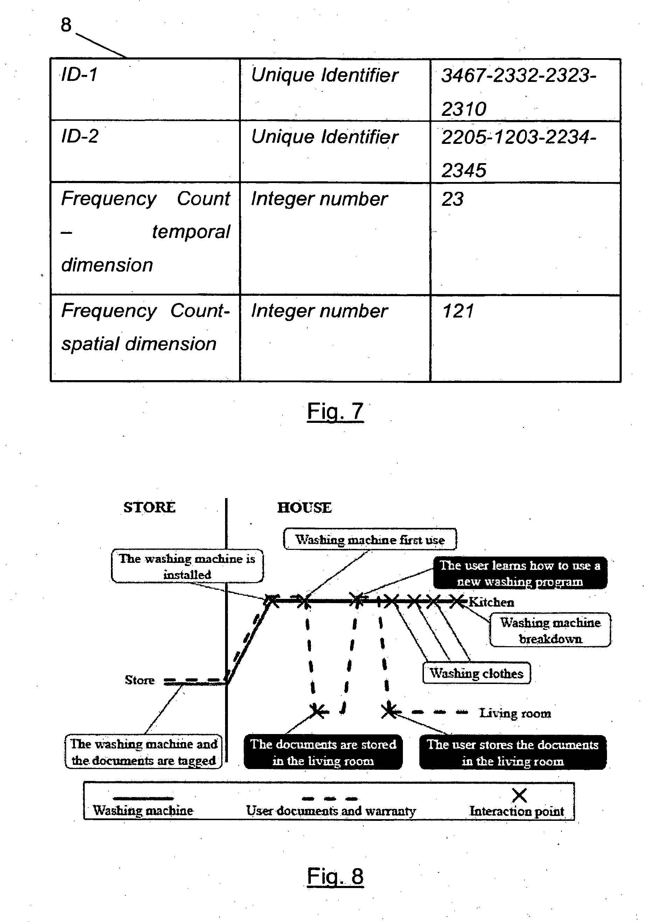

Figure 7 shows an example of information related to affinities between two objects generated by affinity analysis and stored in storage (24). Together with the IDs of both objects (ID-1 and ID-2), a frequency count is kept for the number of spatial affinities detected between those two same objects, and a frequency count is kept for the number of temporal affinities detected between those two same objects.

Figure 10 shows the process followed by a person that wants to receive information about his interactions with an object when he is not able to read the tag (1) attached to the object, for example his car. The process begins when the person uses portable reader (2) to touch a related object (4), for example, the key of the car. Reader (2) reads the ID-1 in the tag (1) and sends it to mobile device (3), this is shown as the filled line in Fig 10. The user then begins a search in mobile device (3) for the objects with affinities with ID-1 stored in storage (24), receiving a list with the ID of those objects (ID-2). One of those objects will be the car in this example, so the user can ask for a service that shows the last interaction (7) with each related object ID-2. After receiving the centroids for each interaction with related objects, mobile device can show the locations of those interactions superimposed over a map. The user will be able to identify his car with this information, or will be able to visit its location shown to find his car.

To limit the number of objects with affinities shown in these services, a threshold value can be selected for the frequency counts.

By selecting a threshold value for the.frequency count of the affinity between two objects, two types of affinity can be distinguished. Sparse affinity when frequency count is less than threshold value. Reinforced affinity when frequency count is greater than threshold value. There is usually a correspondence between a reinforced affinity between two DOIs and a relevant relationship between the two correspondent objects when the user employs them, so services based on affinity can be configured to show only objects with reinforced affinity, limiting the number of objects shown to the user when asking for an affinity service.

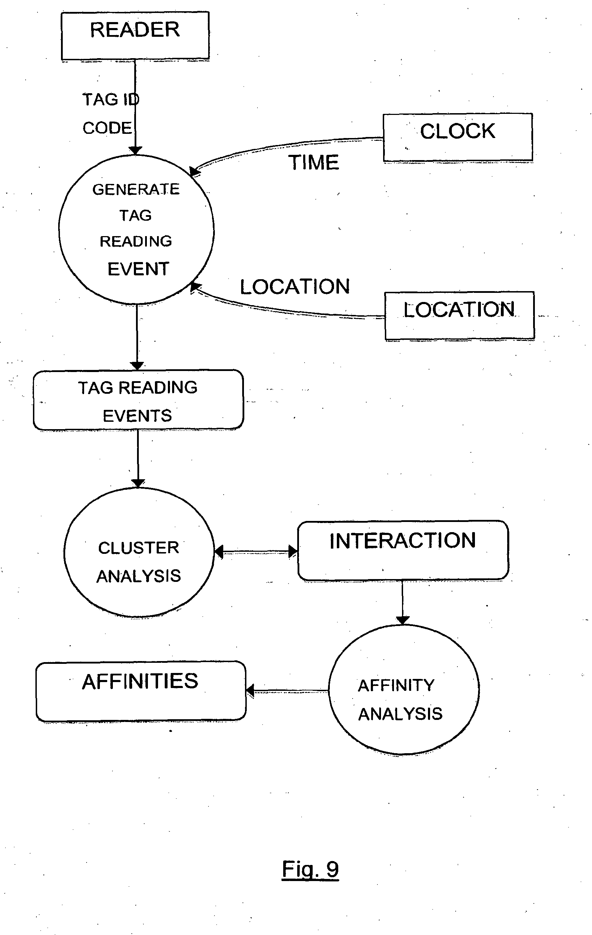

An example of the steps performed by the system to generate interactions (7) and affinities (8) is shown in FIG 9. Portable reader (2) reads an electromagnetic tag (1) and sends its ID to the GENERATE TAG READING EVENT step. This step obtains the TIME and LOCATION from clock (22) and GPS receiver (23) and packs all these data as a tag reading event (5) that is stored in storage (24). Step CLUSTER ANALYSIS reads new tag reading events (5) and looks for an interaction (7) where the tag reading event is near its centroid, based on distance. If the interaction (7) is found, its centroid, duration and spatial extension are updated. If no interaction (7) is found, a new one is created with the tag reading event (5) as centroid and zero value for temporal and spatial extensions. Step AFFINITY ANALYSIS reads new interactions (7) and searches for affinities (8). If an existing affinity (8) is found, its frequency counts are updated. If a new affinity (8) is found, it is created with value one for its frequency counts, and stored in storage (24).

Fig 8 shows a case of use of the proposed invention. The solid line shows the different places where a washing machine is located since production and the dashed line shows the different locations of the user documents and warranty associated to the washing machine. A RF tag is placed in front of the washing machine and the user manual and other documents related like the warranty are also tagged with a standard RF tag. While the washing machine is stored in the warehouse, its tag can be saved in a standard inventory. Then, a user buys this washing machine and takes it to home. Each time the user touches the washing machine (installation, first use...) an interaction point is stored in the user digital memory, where it will also appear the location and the time of the interaction. The user utilization of the user manual and warranty is also saved on the user digital memory, examples of these interaction points are the first use of the washing machine (the user reads the user manuals) and when once the user has learned the correct working of the machine, the documents are stored in some drawer on the living room. The services provided for the system subject of the invention will extrapolate a relationship between the washing machine and the user documents based on similar time and location characteristics on the saved interaction point so when the user demands their digital memory of the user documents the system can show the last stored location of these documents.

## Claims

1. A system of electronically managing interactions of a person with a plurality of objects (4) of his environment, the objects having attached an electromagnetic tag (1) comprising an identification code
further comprising:
- portable reading means (2), to be carried by the person, configured to repeatedly read data of an electromagnetic tag (1) of a plurality of objects (4) when the tag (1) is located within a reading area;
- processing means (3) configured to receive, for each tag (1),
the identification code comprised in the data read by the reading means (2)
with information regarding
the location where,
and the time when, the said tag (1) is read
in order to define a tag reading event (5);
**characterized by** :
- the processing means (3) configured to estimate time interval and location area of an interaction from a plurality of tag reading events (5)
by grouping in a cluster (6) corresponding with the interaction, a plurality of tag reading events (5) with an identical identification code having a deviation between a pair of tag interaction events under a threshold, the deviation being calculated as a difference of values for location and/or for time;
- the processing means (3) further configured to determine a degree of spatial and/or temporal affinity between two interactions based on a common location area (65) and/or simultaneous temporal interval (64) respectively for the clusters (6).

2. The system according to claim 1, wherein the processing means (2) are configured to calculate a set of data for a cluster (6) comprising:
- a temporal boundary as an estimation of time interval (613) of an interaction,
- a spatial boundary as an estimation of location area (612) of an interaction, and
- a centroid for the cluster (6) as a representative point of the interaction.

3. The system according to claim 2, wherein it further comprises storage means (24) configured to store the set of data for the cluster (6).

4. The system according to any of claims 1 to 3, wherein the processing means are configured to register each occurrence of a temporal affinity and/or spatial affinity between the same two objects and to determine the degree of affinity as reinforced affinity when the number of occurrences is above a threshold and, alternatively as sparse affinity when the number of occurrences is below a threshold.

5. The system according to claim 1 to 4, wherein the reading means (2) are coupled with the processing means (3) via a radiofrequency contactless protocol selected at least among:
- Bluetooth,
- IEEE 803.11,
- IrDA,
- Zigbee.

6. The system according to claim 1 to 5, wherein the reading means (2) comprises a reader selected between:
- a RFID reader (11) when the electromagnetic tag (1) is a RFID tag, and
- a NFC reader when the electromagnetic tag (1) is a NFC tag.

7. The system according to any of claims 1 to 6, wherein the electromagnetic tag (1) further comprises spatial and/or temporal information of the object (4) stored thereon.

8. The system according to any of claims 1 to 6, wherein the processing means comprises a GPS receiver to obtain spatial information and/or a clock (22) to obtain temporal information of the read tag (1).

9. The system according to any of claims 1 to 8, wherein the cluster is generated by grouping interaction points based on k-means algorithm.

10. A computer implemented method of electronically managing interactions of a person with a plurality of objects (4) of his environment, the objects having attached an electromagnetic tag (1) comprising an identification code **characterised in that** it comprises the following steps:
- repeatedly reading data of an electromagnetic tag of a plurality of objects (4) when the tag is located within a reading area;
- receiving, for each tag (1), the identification code of the data read with information regarding the location where, and the time when, the said tag (1) is read in order to define a tag reading event (5);
- estimating time interval and location area of an interaction from a plurality of tag reading events (5) by grouping in a cluster (6) corresponding with the interaction, a plurality of tag reading events (5) with an identical identification code having a deviation between a pair of tag interaction events (5) under a threshold, the deviation being calculated as a difference of values for location and/or for time;
- determining a degree of spatial and/or temporal affinity between two interactions based on a common location area (65) and/or simultaneous temporal interval (64) respectively for the clusters (6).

11. The method according to claim 10, wherein the processing step further comprises calculating a set of cluster data comprising a temporal boundary as an estimation of time interval of an interaction, as an estimation of location area of interaction spatial boundary and a centroid for a cluster as a representative point of the interaction.

12. The method according to any of claims 10 to 11, wherein it further comprises registering each occurrence of a temporal affinity and/or spatial affinity between the same two objects and to determine the degree of affinity as reinforced affinity when the number of occurrences is above a threshold and alternatively as sparse affinity when the number of occurrences is below a threshold.

13. Computer program product to perform the steps of any of claims 10 to 12.

## Patentansprüche

1. System zum elektronischen Verwalten von Interaktionen einer Person mit einer Vielzahl an Objekten (4) ihrer Umgebung, wobei an den Objekten ein elektromagnetisches Tag (1) befestigt ist, welches einen Identifizierungscode umfasst, weiterhin umfassend:
- tragbare Lesemittel (2), welche von der Person zu tragen sind, welche ausgebildet sind, um wiederholt Daten eines elektromagnetischen Tags (1) einer Vielzahl an Objekten (4) auszulesen, wenn das Tag (1) innerhalb eines Lesebereichs liegt;
- Verarbeitungsmittel (3), welche ausgebildet sind, um für jedes Tag (1) den Identifizierungscode zu empfangen, welcher in den von den Lesemitteln (2) ausgelesenen Daten enthalten ist, mit Informationen bezüglich
der Lage, in welcher,
und des Zeitpunkts, zu welchem das genannte Tag (1) gelesen wird,
um ein Tag-Lese-Ereignis (5) zu bestimmen;
**dadurch gekennzeichnet, dass**:
- die Verarbeitungsmittel (3) ausgebildet sind, um einen Zeitraum und einen Lagebereich einer Interaktion einer Vielzahl an Tag-Lese-Ereignissen (5) zu schätzen,
indem in einem Cluster (6), welches der Interaktion entspricht, eine Vielzahl an Tag-Lese-Ereignissen (5) mit einem identischen Identifizierungscode gruppiert werden, welcher eine Abweichung zwischen einem Paar an Tag-Interaktions-Ereignissen unter einem Schwellenwert aufweist, wobei die Abweichung als eine Differenz von Werten für die Lage und/oder für den Zeitpunkt berechnet wird;
- die Verarbeitungsmittel (3) weiterhin ausgebildet sind, um jeweils einen Grad an räumlicher und/oder zeitlicher Affinität zwischen zwei Interaktionen auf Grundlage eines gemeinsamen Lagebereichs (65) und/oder eines simultanen Zeitraums (64) für die Cluster (6) zu bestimmen.

2. System nach Anspruch 1, wobei die Verarbeitungsmittel (2) ausgebildet sind, um einen Datensatz für ein Cluster (6) zu berechnen, umfassend:
- eine zeitliche Beschränkung als eine Schätzung eines Interaktionszeitraums (613),
- eine räumliche Beschränkung als eine Schätzung eines Interaktionslagebereichs (612), und
- einen Schwerpunkt für das Cluster (6) als repräsentativen Punkt der Interaktion.

3. System nach Anspruch 2, wobei es weiterhin Speichermittel (24) umfasst, die ausgebildet sind, um den Datensatz für das Cluster (6) zu speichern.

4. System nach einem der Ansprüche 1 bis 3, wobei die Verarbeitungsmittel ausgebildet sind, um jedes Vorkommnis einer zeitlichen Affinität und/oder einer räumlichen Affinität zwischen denselben beiden Objekten zu erfassen und den Affinitätsgrad als verstärkte Affinität zu bestimmen, wenn die Anzahl an Vorkommnissen oberhalb eines Schwellenwerts liegt, und alternativ als geringe Affinität zu bestimmen, wenn die Anzahl an Vorkommnissen unterhalb eines Schwellenwerts liegt.

5. System nach Anspruch 1 bis 4, wobei die Lesemittel (2) mit den Verarbeitungsmitteln (3) über ein kontaktloses Funkfrequenzprotokoll verbunden sind, welches zumindest aus folgenden Protokollen ausgewählt ist:
- Bluetooth,
- IEEE 803.11,
- IrDA,
- Zigbee.

6. System nach Anspruch 1 bis 5, wobei die Lesemittel (2) ein Lesegerät umfassen, welches aus folgenden Lesegeräten ausgewählt ist:
- einem RFID-Lesegerät (11), wenn das elektromagnetische Tag (1) ein RFID-Tag ist, und
- einem NFC-Reader, wenn das elektromagnetische Tag (1) ein NFC-Tag ist.

7. System nach einem der Ansprüche 1 bis 6, wobei das elektromagnetische Tag (1) weiterhin räumliche und/oder zeitliche Informationen des Objekts (4), welche auf diesem gespeichert sind, umfasst.

8. System nach einem der Ansprüche 1 bis 6, wobei die Verarbeitungsmittel einen GPS-Empfänger umfassen, um räumliche Informationen zu erhalten und/oder eine Uhr (22) umfassen, um zeitliche Informationen des Lese-Tags (1) zu erhalten.

9. System nach einem der Ansprüche 1 bis 8, wobei das Cluster durch ein Gruppieren von Interaktionspunkten auf Grundlage eines K-Means-Algorithmus erzeugt wird.

10. Computerimplementiertes Verfahren zum elektronischen Verwalten von Interaktionen einer Person mit einer Vielzahl an Objekten (4) ihrer Umgebung, wobei an den Objekten ein elektromagnetisches Tag (1) befestigt ist, welches einen Identifizierungscode umfasst, **dadurch gekennzeichnet, dass** es folgende Schritte umfasst:
- wiederholtes Auslesen von Daten eines elektromagnetischen Tags einer Vielzahl an Objekten (4), wenn das Tag innerhalb eines Lesebereichs liegt;
- Empfangen, für jedes Tag (1), des Identifizierungscodes der ausgelesenen Daten mit Informationen bezüglich der Lage, in welcher, und des Zeitpunkts, zu welchem das genannte Tag (1) ausgelesen wird, um ein Tag-Lese-Ereignis (5) zu bestimmen;
- Schätzen des Zeitraums und des Lagebereichs einer Interaktion einer Vielzahl an Tag-Lese-Ereignissen (5) durch ein Gruppieren in einem Cluster (6) entsprechend der Interaktion einer Vielzahl an Tag-Lese-Ereignissen (5) mit einem identischen Identifizierungscode, welcher eine Abweichung zwischen einem Paar an Tag-Interaktionsereignissen (5) unterhalb eines Schwellenwerts aufweist, wobei die Abweichung als eine Differenz von Werten für die Lage und/oder für den Zeitpunkt berechnet wird;
- Bestimmen jeweils eines Grads an räumlicher und/oder zeitlicher Affinität zwischen zwei Interaktionen auf Grundlage eines gemeinsamen Lagebereichs (65) und/oder eines simultanen Zeitraums (64) für die Cluster (6).

11. Verfahren nach Anspruch 10, wobei der Bearbeitungsschritt weiterhin das Berechnen eines Satzes von Cluster-Daten umfasst, welches eine zeitliche Beschränkung als eine Schätzung des Zeitraums einer Interaktion, eine räumliche Beschränkung als eine Schätzung des Lagebereichs einer Interaktion und einen Schwerpunkt für ein Cluster als repräsentativen Punkt der Interaktion umfasst.

12. Verfahren nach einem der Ansprüche 10 bis 11, wobei es weiterhin das Erfassen jedes Vorkommnisses einer zeitlichen Affinität und/oder einer räumlichen Affinität zwischen denselben beiden Objekten und das Bestimmen des Affinitätsgrads als verstärkte Affinität, wenn die Anzahl an Vorkommnissen oberhalb eines Schwellenwerts liegt, und alternativ als geringe Affinität, wenn die Anzahl an Vorkommnissen unterhalb eines Schwellenwerts liegt, umfasst.

13. Computerprogrammprodukt zum Ausführen der Schritte eines der Ansprüche 10 bis 12.

## Revendications

1. Système pour gérer électroniquement les interactions d'une personne avec une pluralité d'objets (4) de son environnement, les objets ayant une étiquette électromagnétique (1) insérée qui comprend un code d'identification, comprenant en outre :
- des moyens de lecture mobiles (2), à porter par la personne, configurés pour lire répétitivement les données d'une étiquette électromagnétique (1) d'une pluralité d'objets (4) lorsque l'étiquette (1) est localisée au sein d'une zone de lecture ;
- des moyens de traitement (3) configurés pour recevoir, pour chaque étiquette (1), le code d'identification compris dans les données lues par les moyens de lecture (2) avec des informations sur
la localisation où,
et le moment où, ladite étiquette (1) est lue
afin de définir un événement de lecture d'étiquette (5) ;
**caractérisé en ce que** :
- les moyens de traitement (3) configurés pour estimer l'intervalle de temps et la zone de localisation d'une interaction à partir d'une pluralité d'événements de lecture d'étiquette (5)
en regroupant dans une grappe (6) correspondant à l'interaction, une pluralité d'événements de lecture d'étiquette (5) avec un code d'identification identique ayant un écart entre une paire d'événements d'interaction d'étiquette sous un seuil, l'écart étant calculé comme une différence de valeurs pour la localisation et/ou pour le moment ;
- les moyens de traitement (3) configurés en outre pour déterminer un degré d'affinité spatiale et/ou temporelle entre deux interactions basées respectivement sur une zone de localisation commune (65) et/ou un intervalle temporel simultané (64) pour les grappes (6).

2. Système selon la revendication 1, dans lequel les moyens de traitement (2) sont configurés pour calculer une série de données pour une grappe (6) comprenant :
- une limite temporelle comme une estimation de l'intervalle de temps (613) d'une interaction,
- une limite spatiale comme une estimation de la zone de localisation (612) d'une interaction, et
- un barycentre pour la grappe (6) comme un point représentatif de l'interaction.

3. Système selon la revendication 2, dans lequel il comprend en outre des moyens de stockage (24) configurés pour stocker la série de données pour la grappe (6).

4. Système selon l'une quelconque des revendications 1 à 3, dans lequel les moyens de traitement sont configurés pour enregistrer chaque survenance d'affinité temporelle et/ou affinité spatiale entre les deux mêmes objets et pour déterminer le degré d'affinité comme une affinité renforcée lorsque le nombre de survenances est au-dessus d'un seuil et, alternativement comme une affinité rare lorsque le nombre de survenances est en-dessous d'un seuil.

5. Système selon les revendications 1 à 4, dans lequel les moyens de lecture (2) sont accouplés aux moyens de traitement (3) à travers un protocole sans contact de radiofréquence choisi au moins parmi :
- Bluetooth,
- IEEE 803.11,
- IrDA,
- Zigbee.

6. Système selon les revendications 1 à 5, dans lequel les moyens de lecture (2) comprennent un lecteur choisi entre :
- un lecteur RFID (11) lorsque l'étiquette électromagnétique (1) est une étiquette RFID, et
- un lecteur NFC lorsque l'étiquette électromagnétique (1) est une étiquette NFC.

7. Système selon l'une quelconque des revendications 1 à 6, dans lequel l'étiquette électromagnétique (1) comprend en outre des informations spatiales et/ou temporelles de l'objet (4) stockées dans celle-ci.

8. Système selon l'une quelconque des revendications 1 à 6, dans lequel les moyens de traitement comprennent un récepteur GPS pour obtenir des informations spatiales et/ou une horloge (22) pour obtenir des informations temporelles de l'étiquette lue (1).

9. Système selon l'une quelconque des revendications 1 à 8, dans lequel la grappe est générée par des points d'interaction de regroupement basés sur l'algorithme des k-moyennes.

10. Procédé de mise en oeuvre informatique pour gérer électroniquement les interactions d'une personne avec une pluralité d'objets (4) de son environnement, les objets ayant une étiquette électromagnétique (1) insérée, qui comprend un code d'identification, **caractérisé en ce qu'**il comprend les étapes suivantes :
- lire répétitivement les données d'une étiquette électromagnétique d'une pluralité d'objets (4) lorsque l'étiquette est localisée au sein d'une zone de lecture ;
- recevoir, pour chaque étiquette (1), le code d'identification des données lues avec des informations sur la localisation où, et le moment où, ladite étiquette (1) est lue afin de définir un événement de lecture d'étiquette (5) ;
- estimer l'intervalle de temps et la zone de localisation d'une interaction à partir d'une pluralité d'événements de lecture d'étiquette (5) en regroupant dans une grappe (6) correspondant à l'interaction, une pluralité d'événements de lecture d'étiquette (5) avec un code d'identification identique ayant un écart entre une paire d'évènements d'interaction d'étiquette (5) sous un seuil, l'écart étant calculée comme une différence de valeurs pour la localisation et/ou pour le moment ;
- déterminer un degré d'affinité spatiale et/ou temporelle entre deux interactions basées respectivement sur une zone de localisation commune (65) et/ou un intervalle temporel simultané (64) pour les grappes (6).

11. Procédé selon la revendication 10, dans lequel l'étape de traitement comprend en outre le calcul d'une série de données de grappe comprenant une limite temporelle comme une estimation de l'intervalle de temps d'une interaction, comme une estimation de la zone de localisation d'une limite spatiale d'interaction et un barycentre pour une grappe comme un point représentatif de l'interaction.

12. Procédé selon l'une quelconque des revendications 10 à 11, dans lequel il comprend en outre l'enregistrement de chaque survenance d'une affinité temporelle et/ou d'une affinité spatiale entre les deux mêmes objets et pour déterminer le degré d'affinité comme une affinité renforcée lorsque le nombre de survenances est au-dessus d'un seuil et alternativement comme une affinité rare lorsque le nombre d'occurrences est en-dessous d'un seuil.

13. Produit de programme informatique pour réaliser les étapes de l'une quelconque des revendications 10 à 12.
